# EUROPEAN PATENT SPECIFICATION

(11) **EP 1 886 638 B1**
(45) Date of publication and mention of the grant of the patent: **03.12.2008**
(21) Application number: 06015460.6
(22) Date of filing: 25.07.2006
(51) Int. Cl.: A61B 19/00

(54) **Apparatus for use in medical treatment**
Gerät zur Anwendung in einer medizinischer Behandlung
Dispositif a usage médical

(43) Date of publication of application: 13.02.2008
(73) Proprietor: Widgerow, Alain David, Prof., Gallo Manor Sandton; Guateng (ZA)
(72) Inventor: Widgerow, Alain David, Prof., Gallo Manor Sandton; Guateng (ZA)
(74) Representative: Galgut, John

(56) References cited:
- WO-A-01/34220
- WO-A-01/93771
- WO-A-96/12518
- FR-A1- 2 615 397
- US-A- 5 549 672

## Description

### FIELD OF THE INVENTION

This invention relates to methods of and apparatus for use in medical treatment.

After certain surgical procedures it is necessary to insert prostheses under the skin. However, there are often difficulties because the skin is tight and unable to stretch to accommodate the prosthesis. The procedure presently adopted to meet this problem is to insert a hollow flexible expander under the skin and to increase the volume of the expander by introducing a liquid into it and thereby stretching the skin. The procedure continues until the skin is large enough to accommodate the prosthesis there below with the patient remaining comfortable. Typical examples dsicussing this procedure are disclosed in WO 01/93771 and WO 96/9612518 which will be discussed more fully below. The prosthesis is then permanently inserted in position. The same procedure may be adopted to repair skin on the head, neck, hands, arms and legs where, although a prosthesis is not used, skin has to be stretched to replace damaged or removed skin. It will be understood that in certain circumstances, other tissue may also have to be stretched/expanded and the term "skin" as used herein is intended to cover such tissue, unless clear from the context.

In known procedures, the expander is inserted under the skin and a permanently attached flexible tube is usually buried under the skin. A sealed receiver unit/valve, which is covered by a membrane, is connected to the distal end of the flexible tube and buried beneath the skin or may be integrated into the expander. The patient now visits a medical practitioner, normally a surgeon, who introduces the liquid into the expander through the valve beneath the skin thereby enlarging it to stretch the skin. This the medical practitioner does by using a fine needle attached to a syringe to pierce the skin and valve membrane and to feed liquid into the receiver unit valve from whence it passes via the flexible tube into the expander. It will be understood that the stretching of the skin must take place in small increments. If the skin is stretched to too great an extent, the patient will suffer discomfort or pain, and in extreme cases the scar tissue may tear. This limits the amount of expansion of the expander during each visit to the medical practitioner who has to expend considerable care and expertise in feeding liquid to the expander. Consequently the treatments required which normally take place on a weekly basis, are numerous, and, for example, in the case of stretching skin after a mastectomy, the procedures may extend over a period of as long as three months. Furthermore, of course, the procedure is expensive and inconvenient requiring the patient to visit the practitioner (usually necessitating travel, waiting in the reception room etc). In addition until the reconstructive surgery takes place the patient will suffer from abnormal appearance due to the expansion as aforesaid. There will also be increased risk of complications where the expansion takes place over an extended period of time. For all these reasons it is desirable for the expansion period to be decreased which would contribute to patient convenience, decreased costs and improved acceptance of this technique.

In WO 01/93771 there is described apparatus for use in expanding skin, comprising an inflatable expander that is capable of being introduced under the skin of a patient which is required to be stretched, a flexible tube leading from the expander and being capable of passing through the skin of the patient, an interconnector attached to the tube outside the body of the patient, and a liquid delivery machine connectable to the interconnector, the machine including a pump having a pump chamber, an inlet port leading to the pump chamber, flow control means for controlling flow into the pump chamber, an outlet port frokm the pump chamber leading to a non-return valve. In this arrangement the pump is a motor driven rotary pump. The pump is controlled by software and/or a pressure sensor determining the amount of liquid to be pumped to the expander. It is a complex unit which appears to be difficult to operate. Although in the specitication it is stated that this apparatus can be used by a patient, the apparatus does not seem to be very user frreiendly and is more likely to have

WO 96/12518 shows a syringe operated unit for injecting liquid into body structures for augmenting volumentrically deficient structures or for carrying out similar actions. This apparatus appears to be intended to be operated by medical personnel.

### SUMMARY OF THE INVENTION

According to one aspect of the invention there is provided apparatus for use in expanding skin, comprising an inflatable expander that is capable of being introduced under the skin of a patient which is required to be stretched, a flexible tube leading from the expander and being capable of passing through the skin of the patient, an interconnector attached to the tube outside the body of the patient, and a liquid delivery machine connectable to the interconnector, the machine including a pump having a pump chamber, an inlet port leading to the pump chamber, flow control means for controlling flow into the pump chamber, an outlet port from the pump chamber leading to a non-return valve characterised in that the liquid delivery machine comprises a cylinder, a piston within the cylinder movable from an initial position in which the volume of the cylinder is at a maximum and a depressed position in which the volume of the cylinder is at a minimum, manually operable operating means which is manually operable to move the piston from the initial position to the depressed position, and spring means for biasing the piston to the initial position, and in that the strength of the spring means and the effect of the flow control means is such that a substantial time, for example of the order of passes before the piston is moved from the depressed position to the initial position.

Normally a patient using this apparatus will not deliver too much liquid by means of the liquid delivery machine because discomfort and pain will prevent such excessive delivery. Preferably, however, the liquid delivery machine incorporates means for ensuring that only limited amounts of liquid such as can be dispensed thereby during a specified time period.

Preferably connector means are provided between the interconnector and the dispensing end of the liquid delivery machine, which are easily cleanable so that the patient may maintain the apparatus sterile. A covering is normally provided for the interconnector, so that this is protected when not in use. Similarly, a covering member may be provided for the dispensing end of the liquid delivery machine.

An embodiment of the invention will now be described by way of example, with reference to the accompanying drawings.

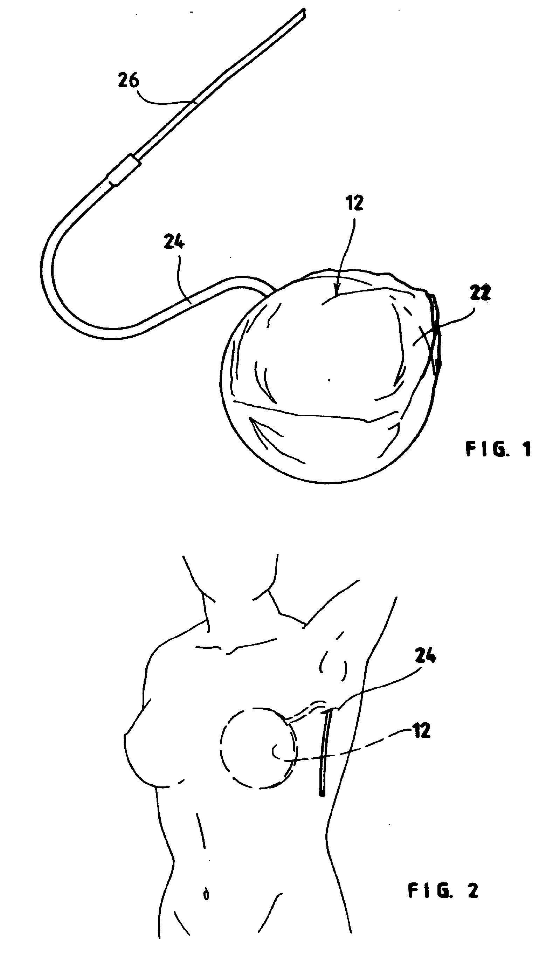

In the drawings:-
- Figure 1: is a perspective view of an expander of the invention, incorporating a trocar,
- Figure 2: is a diagrammatic view showing the position of the expander applied to a patient who has had a mastectomy,

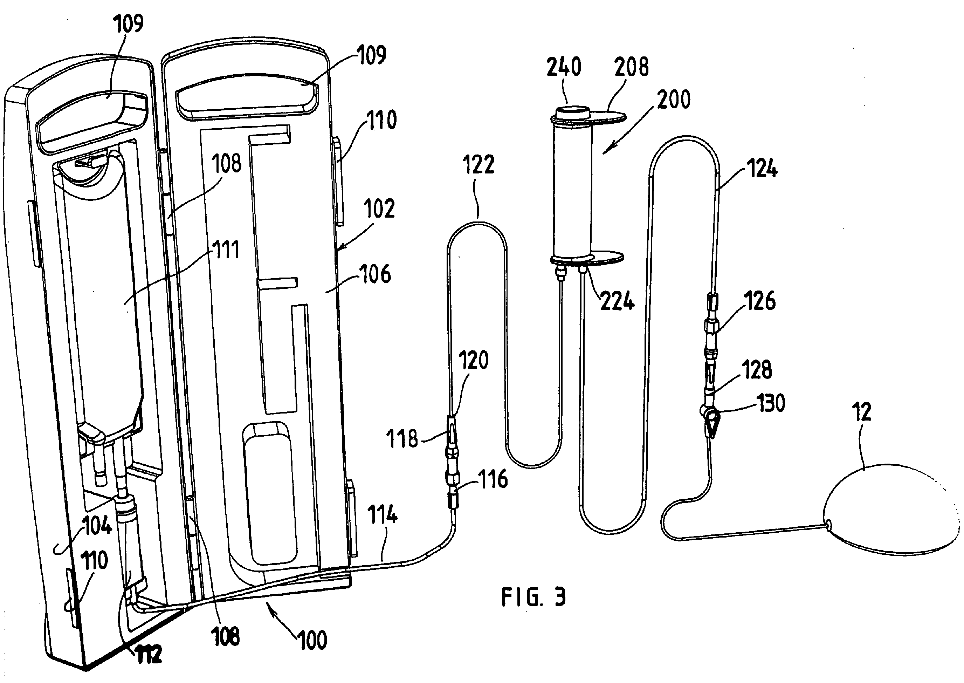

- Figure 3: is a view of a PCD machine with the container in the open position,
- Figure 4: shows the container closed,

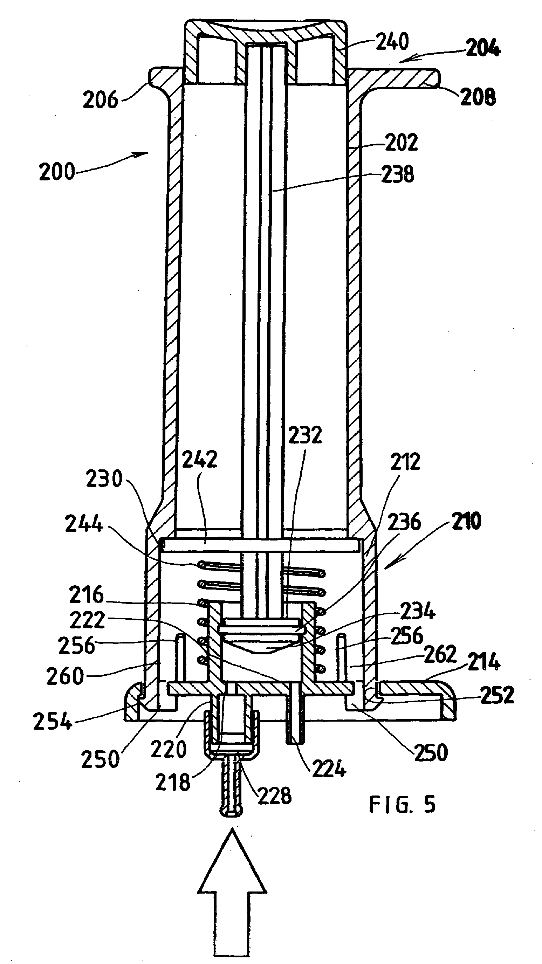

- Figure 5: is a section on line 5-5 of Figure 3, and

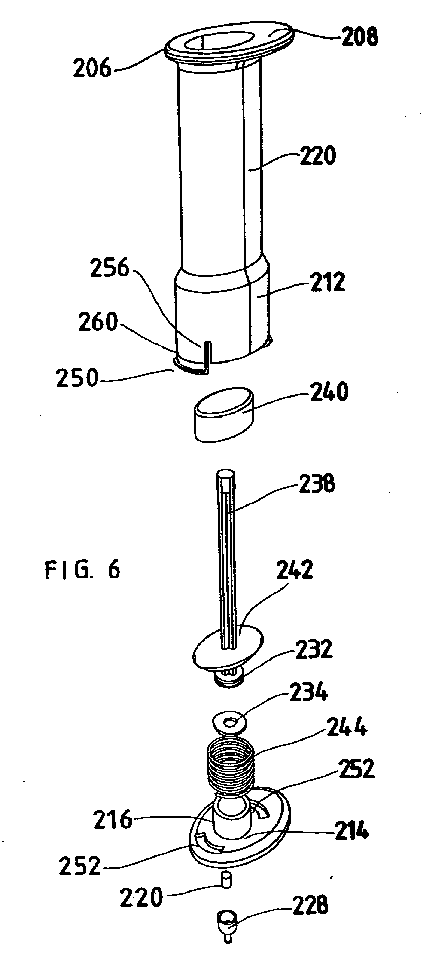

- Figure 6: is an exploded view of the PCD machine.

Referring now to the drawings, apparatus 10 of the invention comprises an expander 12, an intermediate connector set 14 and a patient controlled liquid delivery machine ("PCD machine") 100 tube described more fully below together with a connector pipe 18.

The expander 12 comprises a hollow plastic member having a reasonably rigid circular base 20 and a collapsible part spherical top part 22 formed of flexible and expandable plastic material. A permanently connected flexible silicone tube 24 is connected to the expander 12, adjacent to the base 20. At its free end, the tube 24 is connected to a trocar 26. When a liquid is introduced into the expander 12, as will be described below, the volume of the expander increases and thus stretches the skin covering the expander 12.

The PCD machine 100 has a housing 102 comprising two members 104 and 106 which are both plastics mouldings and which are connected together by hinge members 108. The interior of the members 104 and 106 are suitably shaped to provide recesses for the parts that will be described below so that when closed there will be a compact unit which a patient can carry and store as desired. A transverse opening 109 passes through the upper ends of the members 104 and 106 to form a handle to facilitate carrying the housing 102. Suitable closure means 110 are provided to hold the members together when the housing is closed.

Within one member 104 is carried a bag 111 containing saline and connected to a suitable filter mechanism 112 as is well known in the medical profession. The mechanism 112 leads to a narrow tube 114 which terminates in a one way valve with an antibacterial filter 116 and having a male luer connector 118. A female luer connector 120 is connected by a silicon tube 122 to the inlet duct of a pump 200 (to be described below) the outlet duct of which leads via a silicon tube 124 to another one way valve 126 incorporating an antibacterial filter and having a male luer connector 128 which may connect to junction 130 incorporating a lever operated control valve that in turn is connected to the tube leading to the expander 12. This junction 130 serves as an interconnector to the tube 24.

The pump 200 comprises an enlarged hollow gripping part 202 that is oval in section. It has an open top end 204 which is flanged and the flange 206 has an enlarged part 208 at one of its smaller sides. At its lower end 210 is an enlarged portion 212 mounted on a bottom cross plate 214. On the cross plate 214 there is centrally mounted a short cylinder 216 forming a pump chamber as will become apparent. At its lower end the cylinder 216 has an inlet port 218 passing through the cross plate 214 and being connected to a short connector tube 220 and an outlet port 222 also passing through the cross plate 214 and being connected to an outlet tube 224. The inlet tube 220 carries a union 226 within which is a control valve or flow limiter 228.

Internally at the juncture of the gripping part 202 and the enlarged portion 212 is a shoulder 230 facing the cross plate 214. A piston member 232 is mounted in the cylinder 216 being sealed thereto by a suitable cap seal 234 with an enlarged ring surround 236. The piston member 232 is movable from an initial position in which the volume of the cylinder 216 is at a maximum, to a depressed position in which the volume of the cylinder is at a minimum. The piston member 232 is carried at the lower end of an elongated rod 238 that is cruciform in section. A smooth sided operating button 240 is provided at the upper end of the rod 238 and this projects slightly above the flange 206. A guide member 242 is mounted on the rod 238 within the enlarged portion and is slidably mounted therein from an upper position in which it butts against the shoulder 230 and a lower position determined by the engagement of the piston member 232 against the part of the cross plate 214 forming the bottom of the cylinder 216. By depressing the button 240 the user moves the piston from initial position to the depressed position. A spring 244 biasses the guide member 242 towards and against the shoulder 230 and in moving to this position, the guide member moves the button from the depressed position to the initial position. The dimensions of the cylinder 216 and the stroke of the piston member 232 is such that on each operation about 2 mℓ liquid is pumped. The flow limiter 228 determines the speed that liquid can enter the cylinder 216 below the piston member 232, it being noted that significant amounts of liquid cannot be drawn in through the outlet 224 and tube 124 because of the provision of the one way valve 126. This coupled with the strength of the spring 244 determines the time taken for the piston member to move to the initial position so that the button 242 will be in position to be depressed. The device is pre-set so that cylinder 216 will be refilled only after a predetermined and relatively substantial time which may conveniently be between five and ten minutes. Thus only limited amounts of liquid can be dispersed during any specified time period.

The gripping part 202 is connected to the cross plate 214 by means of extension pieces 250 that pass through arcuate slots 252 in the cross plate 214 and have outwardly projecting flange portions 254 that lie under the cross plate 214. The enlarged portion 212 has two pairs of vertical slots 256 and 258 which form resilient arms 260 and 262 that carry the extension pieces 250 and that can be pivoted inwardly to permit the flange portions 254 to pass through the slots 252 and when released the arms 260 and 262 move outwardly so that the flange portions lie under the cross plate 214 to hold the gripping part thereto. Thus the pump 200 can be disassembled for cleaning and sterilizing and for adjusting the volume of liquid expressed.

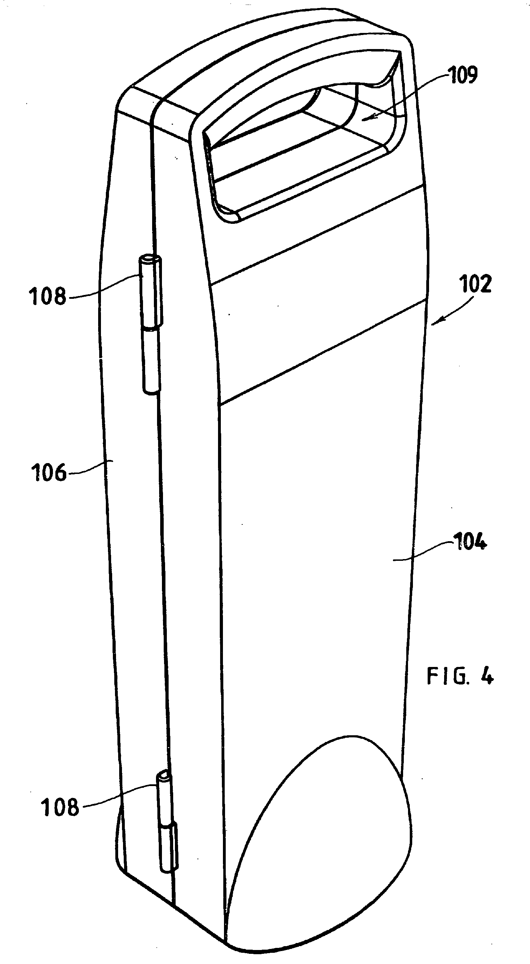

In use, after a mastectomy, the expander 12, which is in the collapsed condition, is surgically fitted under the skin retropectorally on the mastectomy side in the normal fashion (see Figure 4). By means of the trocar 26 an opening is made in the skin, preferably below the arm pit, so that the tube 24 is exposed to the external atmosphere. The trocar 26 is now removed by cutting the end part of the tube 24. The junction 130 is connected to the free end of the tube 24. An amount of liquid is introduced into the expander by the surgeon. The control valve 132 is closed, and if desired a cap or other cover is placed over the junction. The exposed part of the tube 24 is bound to the patient in a way to protect this item and to cause the patient minimum discomfort.

After a period of about 1 - 5 days after the implant procedure (or sooner in some cases when the surgeon believes applicable) the patient may now commence expansion.

The patient will now use the housing 100 and will add a fresh bag of saline 111. By means of the one way valve 116 the Luer connectors 118 and 120 the bag is connected to the pump 200. The patient will prime the pump by pressing the operating button 240 a few occasions until she sees that liquid is discharged from the outlet filter 126. The patient then fits the connector 128 to the control valve. By depressing the button 240 at this stage the patient forces about 2 ml liquid into the expander. This may be adequate at this stage. The patient when comfortable may wish to depress the button 240 again to expand the expander. However, as mentioned above the pump remains inactive for ten minutes (thus the patient will not overfill the expander). The patient can then insert further amounts of saline into the expander as desired but ensuring that there is not so much inserted as to cause discomfort. By increasing the expander size with the small amounts of liquid at reasonably spaced intervals the skin will stretch without undue pain or discomfort.

Because the button is smooth and projects only a small amount from the top of the gripping part the patient cannot hold this and pull on it to supplement the effect of the spring 244 to cause the cylinder to be recharged more quickly than the time that has been set. By depressing the button 240, the patient ensures that a preset amount of liquid is introduced into the expander 12. The PCD machine 100 prevents excessive introduction of liquid into the expander 12 during each procedure. However, the procedure can be repeated multiple times a day so that the skin is stretched to a small extent after each procedure. After each stretching operation the skin relaxes, so that during the next stretching operation the skin will be ready to stretch further and pain will be reduced.

The patient may apply topical creams to the skin being stretched to facilitate the process of stretching of the skin and to ease any pain involved. It is desirable that the surgeon or the medical practitioner monitors the process once or twice weekly which may or may not involve visits to the surgeon or medical practitioner. The process is continued until the breast is slightly over expanded which should be determined by the surgeon and is left as such until the next stage is scheduled. The expander is then removed and the normal routine of the breast reconstruction is continued

We have found that it is relatively easy to instruct the patient how to operate and refill the PCD and how to keep the various parts clean and sterile. It is also easy to disassemble the PCD machine for cleaning and sterilisation.

We have found that because the expansion takes place regularly with very small increments, it is possible to complete the full expansion of the expander in a very short period of time such as typically three weeks. Thus the reconstruction time can be decreased by about two months or more. Further as the visits to the medical practitioner can be reduced significantly the cost of the procedure will also be considerably reduced.

The invention is not limited to the precise details hereinbefore described and illustrated. For example the same procedure may be adopted for any case of tissue expansion e.g. to repair skin on the head, neck, hands, arms and legs where, although a prosthesis is not used, skin has to be stretched to replace damaged or removed skin. The shape of the expander would vary according to the needs of the reconstruction. The expander may vary as will be appreciated by those skilled in this art. The use of procedure on the skin on the head has a benefit in that hair will grow through this skin which usually does not occur where skin is taken from another part of the patients body. The amount of liquid provided on each operation of the PCD machine may vary as desired although this usually no more than 1-5 millilitres of liquid being introduced. A non-return valve may be provided at the outlet tube. Other liquids may be used to enlarge the expander.

In a further modification pressure reading monitors may be attached to the expander tube enabling the monitoring of pressure transmitted within the expander and on to the overlying skin.

Future scenarios are envisaged where teletype monitoring from home is possible, whereby photo or video documentation of the status of the expansion is transmitted to the surgical control centre for monitoring by medical personnel, e.g. a surgeon, a doctor or specially trained nurse.

## Claims

1. Apparatus for use in expanding skin, comprising
an inflatable expander (12) that is capable of being introduced under the skin of a patient which is required to be stretched,
a flexible tube (24) leading from the expander and being capable of passing through the skin of the patient,
an interconnector(130) attached to the tube outside the body of the patient, and
a liquid delivery machine connectable to the interconnector, the machine including a pump (200) having
a pump chamber (216),
an inlet port (218) leading to the pump chamber (216), and
an outlet port (224) from the pump chamber (216) leading to a non-return valve (126) and thence to the expander (12),
**characterised in that** the liquid delivery machine comprises
a cylinder (216) forming the pump chamber,
flow control means (228) for controlling flow into the pump chamber,
a piston (232) within the cylinder (216) movable from an initial position in which the volume of the cylinder is at a maximum and a depressed position in which the volume of the cylinder is at a minimum,
manually operable operating means (240) which is manually operable to move the piston (232) from the initial position to the depressed position, and
spring means (244) for biasing the piston (232) to the initial position,
and **in that** the strength of the spring means (244) and the effect of the flow control means (228) is such that a substantial time passes before the piston is moved from the depressed position to the initial position.

2. Apparatus as claimed in claim 1 **characterised in that** the said substantial amount of time is between five to ten minutes.

3. Apparatus as claimed in claim 1 or 2 **characterised in that** the liquid delivery machine incorporates means for ensuring that only a limited amount of liquid can be dispensed thereby during a specified time period.

4. Apparatus as claimed in claim 3 **characterised in that** the said limited amount of liquid comprises 2ml liquid.

5. Apparatus as claimed in any one of the preceding claims **characterised in that** the operating means comprises a button (240) which is connected to the piston (232) by a connecting device (238).

6. Apparatus as claimed in claim 5 **characterised in that** the cylinder (216) is shaped to be able to be gripped manually and has the button (240) protruding from one end thereof so that when the cylinder is gripped a patient can depress the button by means of the patient's thumb.

7. Apparatus as claimed in claim 6 **characterised in that** the button (240) projects only a small distance from the cylinder (216) and is smooth-sided so that the patient cannot grip the button to speed the movement of the piston to the initial position.

8. Apparatus as claimed in any one of the preceding claims **characterised in that** a casing (100) is provided for all the parts which will enable the patient to move the device conveniently to any suitable place for use.

## Patentansprüche

1. Vorrichtung zur Verwendung zur Ausdehnung von Haut, umfassend:
einen aufblasbaren Dehner (12), welcher geeignet ist, um unter die Haut eines Patienten, welche gestreckt werden soll, eingeführt zu werden,
eine flexible Röhre (24), welche von dem Dehner führt, und geeignet ist, um durch die Haut des Patienten zu führen,
eine Zwischenverbindung (130), welche außerhalb des Körpers des Patienten an dem Rohr befestigt ist, und
eine Flüssigkeitsliefermaschine, welche mit der Zwischenverbindung verbindbar ist, wobei die Maschine eine Pumpe (200) umfasst, welche auf weist:
- eine Pumpkammer (216),
- einen Einlassanschluss (218), welcher zu der Pumpkammer (216) führt, und
- einen Auslassanschluss (224) von der Pumpkammer (216), welcher zu einem Rücklaufsperreventil (126) und daher zu dem Dehner (12) fuhrt,
**dadurch gekennzeichnet, dass** die Flüssigkeitsliesefermaschine umfasst:
einen Zylinder (216), welcher die Pumpkammer bildet,
Flusssteuermittel (228) zum Steuern eines Flusses in die Pumpkammer,
einen Kolben (232) in dem Zylinder (216), welcher von einer Startstellung, in welcher das Volumen des Zylinders maximal ist, und einer herab gedrückten Stellung, in welcher das Volumen des Zylinders minimal ist, bewegbar ist,
ein manuell bedienbares Bedienmittel (214), welches manuell bedienbar ist, um den Kolben (232) von der Startstellung in die herab gedrückte Stellung zu bewegen, und
Federmittel (244) zum Vorspannen des Kolbens (232) in der Startstellung,
und dadurch, dass die Stärke des Federmittels (244) und die Einwirkung des Flusskontrollsteuerungsmittels (228) so ist, dass das eine merkliche Zeit vergeht, bevor der Kolben von der herab gedrückten Stellung in die Startstellung bewegt wird.

2. Vorrichtung nach Anspruch 1, **dadurch gekennzeichnet, dass** der merkliche Zeitraum zwischen fünf und zehn Minuten beträgt.

3. Vorrichtung nach Anspruch 1 oder 2, **dadurch gekennzeichnet, dass** die Flüssigkeitsliefermaschine Mittel zum Gewährleisten einschließt, dass damit nur eine begrenzte Menge von Flüssigkeit während eines vorgegebenen Zeitraums abgegeben werden kann.

4. Vorrichtung nach Anspruch 3, **dadurch gekennzeichnet, dass** die begrenzte Flüssigkeitsmenge 2 Milliliter Flüssigkeit umfasst.

5. Vorrichtung nach irgendeinem der vorhergehenden Ansprüche, **dadurch gekennzeichnet, dass** das Bedienmittel einen Knopf (240) umfasst, welcher mit dem Kolben (232) durch eine Verbindungsvorrichtung (238) verbunden ist

6. Vorrichtung nach Anspruch 5, **dadurch gekennzeichnet dass** der Zylinder (216) geformt ist, um manuell erfasst zu werden, und den Knopf (240) aufweist, welcher von dem einen Ende davon herausragt, so dass, wenn der Zylinder gefasst wird, ein Patient den Knopf mittels dem Daumen des Patienten herunter drücken kann.

7. Vorrichtung nach Anspruch 6, **dadurch gekennzeichnet, dass** der Knopf (240) nur eine kleine Strecke von dem Zylinder (216) herausragt und flachseitig ist, so dass der Patient den Knopf nicht fassen kann, um die Bewegung des Kolbens in die Startstellung zu beschleunigen.

8. Vorrichtung nach irgendeinem der vorhergehenden Ansprüche, **dadurch gekennzeichnet, dass** ein Gehäuse (100) für alle Teile bereitgestellt wird, was dem Patienten ermöglicht, die Vorrichtung bequem an jedem geeigneten Ort zur Benutzung zu bewegen.

## Revendications

1. Dispositif à utiliser pour étendre la peau, comprenant :
un ballon d'extension gonflable (12) qui peut être introduit sous la peau d'un patient qui doit être étirée,
un tube flexible (24) partant du ballon d'extension et pouvant traverser la peau du patient,
un dispositif d'interconnexion (130) fixé au tube à l'extérieur du corps du patient, et
une machine de distribution de liquide pouvant se brancher au dispositif d'interconnexion, la machine comprenant une pompe (200) comportant :
une chambre de pompe (216),
un orifice d'entrée (218) menant à la chambre de pompe (216), et
un orifice de sortie (224) de la chambre de pompe (216) menant à une vanne antiretour (126) et de là au ballon d'extension (12),
**caractérisé en ce que** la machine de distribution de liquide comprend :
un cylindre (216) formant la chambre de pompe,
des moyens de régulation d'écoulement (228) pour réguler l'écoulement dans la chambre de pompe,
un piston (232) à l'intérieur du cylindre (216), mobile entre une position initiale, dans laquelle le volume du cylindre se trouve à un maximum, et une position enfoncée, dans laquelle le volume du cylindre se trouve à un minimum,
des moyens d'actionnement (240) à usage manuel qui sont utilisables manuellement pour déplacer le piston (232) de la position initiale à la position enfoncée, et
des moyens de ressort (244) pour solliciter le piston (232) vers la position initiale,
et **en ce que** la force des moyens de ressort (244) et l'effet des moyens de régulation d'écoulement (228) sont tels qu'un temps substantiel s'écoule avant que le piston ne soit déplacé de la position enfoncée jusqu'à la position initiale.

2. Dispositif selon la revendication 1, **caractérisé en ce que** ladite quantité substantielle de temps se situe entre cinq et dix minutes.

3. Dispositif selon la revendication 1 ou 2, **caractérisé en ce que** la machine de distribution de liquide intègre des moyens pour assurer que seule une quantité limitée de liquide peut être distribuée par celle-ci pendant une période de temps spécifiée.

4. Dispositif selon la revendication 3, **caractérisé en ce que** ladite quantité limitée de liquide comprend 2 ml de liquide.

5. Dispositif selon l'une quelconque des revendications précédentes, **caractérisé en ce que** les moyens d'actionnement comprennent un bouton (240) qui est relié au piston (232) par un dispositif de liaison (238).

6. Dispositif selon la revendication 5, **caractérisé en ce que** le cylindré (216) est formé pour pouvoir être saisi manuellement et le bouton (240) en faisant saillie à partir d'une extrémité de celui-ci de sorte que, quand le cylindre est saisi, un patient peut appuyer sur le bouton au moyen de son pouce.

7. Dispositif selon la revendication 6, **caractérisé en ce que** le bouton (240) fait saillie seulement sur une petite distance à partir du cylindre (216) et présente des côtés lisses de sorte que le patient ne peut pas saisir le bouton pour accélérer le mouvement du piston jusqu'à la position initiale.

8. Dispositif selon l'une quelconque des revendications précédentes, **caractérisé en ce qu'**un coffret (100) est prévu pour toutes les pièces, qui permettra au patient de déplacer le dispositif de façon pratique vers un endroit quelconque adapté à l'utilisation.
